# EUROPEAN PATENT APPLICATION

(11) **EP 4 497 324 A1**
(43) Date of publication of application: **29.01.2025**
(21) Application number: 23187535.2
(22) Date of filing: 25.07.2023
(51) Int. Cl.: A01G 18/00, A01G 31/00, A01K 31/00, A01K 61/00

(54) **CONFIGURATION SYSTEM FOR GREEN ENERGY AGRICULTURAL PARK**

(71) Applicant: Season Agricultural Technology Co., Ltd., Tainan City 711011 (TW)
(72) Inventor: YANG, Ching-Chieh, 711011 Tainan City (TW)
(74) Representative: Heisel, Wolfgang

(57) **Abstract**

A configuration system for a green energy agricultural park includes a mushroom cultivation section (10), a super-intensive breeding section (20), a non-toxic vegetable planting section (30), a poultry breeding section (40), a CAS cold chain section (50), a black soldier fly breeding section (60), a goods collection sales section (70), an electric vehicle charging section (80), and a green energy power generation section (90). The green energy power generation section generates electricity and supplies an electric power to the mushroom cultivation section, the super-intensive breeding section, the non-toxic vegetable planting section, the poultry breeding section, the CAS cold chain section, the black soldier fly breeding section, the goods collection sales section, and the electric vehicle charging section. The black soldier fly breeding section breeds black soldier flies to eat agricultural organic resources produced in the green energy agricultural park and to generate black soldier fly manure.

## Description

The present invention relates to a system and, more particularly, to a configuration system for a green energy agricultural park.

In recent years, the concept of sustainable management of enterprises has risen, so that the revenue, profit, employee benefits, and shareholders' rights and interests of enterprises have become basic items of evaluation. Thus, the ESG score is often served as the important indicators and investment decisions for sustainable development of a company. The ESG scores are mainly divided into three major categories, including environmental management, social responsibility, and corporate governance. Many companies mainly focus on the environmental management, such as forestation, green electricity certificates, replacement of LED lamps, etc., to reduce the carbon emissions. However, the amount of carbon reduction is quite limited and is of little benefit to the overall ESG score.

In accordance with the present invention, there is provided a configuration system for a green energy agricultural park, comprising a mushroom cultivation section for planting mushrooms, a super-intensive breeding section for breeding aquatic products, a non-toxic vegetable planting section for planting pesticide-free and organic vegetables, a poultry breeding section for breeding poultry, a CAS cold chain section for processing, packaging, and cold-chain storing agricultural organic matters produced in the mushroom cultivation section, the super-intensive breeding section, the non-toxic vegetable planting section, and the poultry breeding section, a black soldier fly breeding section for breeding black soldier flies which eat agricultural organic resources produced in the mushroom cultivation section, the super-intensive breeding section, the non-toxic vegetable planting section, the poultry breeding section, and the CAS cold chain section, a goods collection sales section for serving as a selling shop of agricultural products produced in the mushroom cultivation section, the super-intensive breeding section, the non-toxic vegetable planting section, the poultry breeding section, and the CAS cold chain section, and for collecting and transporting the agricultural products, an electric vehicle charging section for installing charging stations to charge electric cars and motorcycles, and a green energy power generation section for generating electricity and supplying an electric power to the mushroom cultivation section, the super-intensive breeding section, the non-toxic vegetable planting section, the poultry breeding section, the CAS cold chain section, the black soldier fly breeding section, the goods collection sales section, and the electric vehicle charging section.

According to the primary advantages of the present invention, the configuration system for a green energy agricultural park greatly reduces the carbon emission, produces non-toxic agricultural products, regularly gives back to local farmers and to provide fertilizers, and brings an outside income to the enterprise. Thus, the present invention satisfies requirements of the essential points, including environmental management, social responsibility, and corporate governance, in the ESG score, and it is believed that the present invention will get a good evaluation in the ESG score, which is beneficial to the sustainable development of enterprises.

In the drawing:

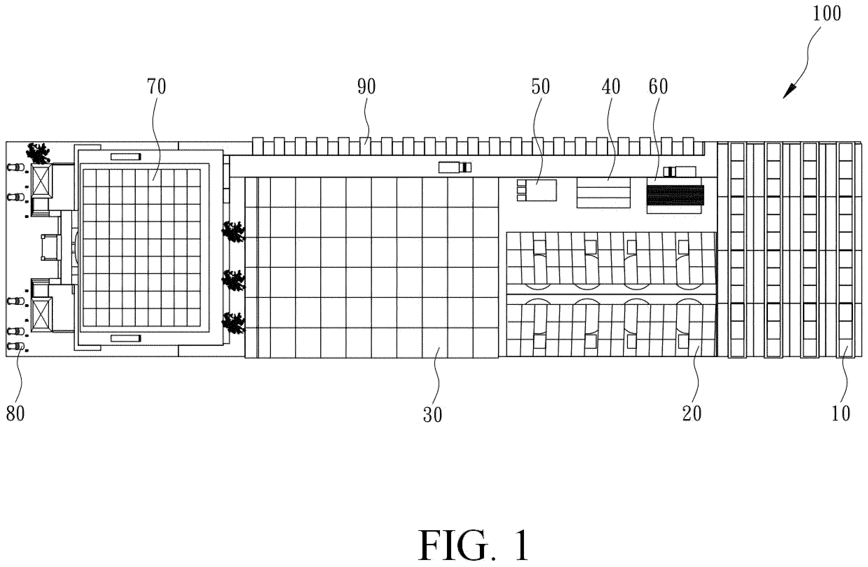

FIG. 1 is a schematic layout of a configuration system for a green energy agricultural park in accordance with the preferred embodiment of the present invention.

Referring to FIG. 1, a configuration system 100 for a green energy agricultural park (or garden) in accordance with the preferred embodiment of the present invention comprises a mushroom cultivation section (or area) 10 for planting mushrooms, a super-intensive breeding section 20 for breeding aquatic products, a non-toxic vegetable planting section 30 for planting pesticide-free and organic vegetables, a poultry breeding section 40 for breeding poultry, a CAS (certified agriculture standard) cold chain section 50 for processing, packaging, and cold-chain storing agricultural organic matters produced in the mushroom cultivation section 10, the super-intensive breeding section 20, the non-toxic vegetable planting section 30, and the poultry breeding section 40, a black soldier fly (or Hermetia illucens) breeding section 60 for breeding black soldier flies which eat agricultural organic resources (or residuals) produced in the mushroom cultivation section 10, the super-intensive breeding section 20, the non-toxic vegetable planting section 30, the poultry breeding section 40, and the CAS cold chain section 50, a goods collection sales section 70 for serving as a selling shop of agricultural products produced in the mushroom cultivation section 10, the super-intensive breeding section 20, the non-toxic vegetable planting section 30, the poultry breeding section 40, and the CAS cold chain section 50, and for collecting and transporting the agricultural products, an electric vehicle charging section 80 for installing charging stations (or piles) to charge electric cars and motorcycles, and a green energy power generation section 90 for generating electricity and supplying an electric power to the mushroom cultivation section 10, the super-intensive breeding section 20, the non-toxic vegetable planting section 30, the poultry breeding section 40, the CAS cold chain section 50, the black soldier fly breeding section 60, the goods collection sales section 70, and the electric vehicle charging section 80.

In the preferred embodiment of the present invention, the mushroom cultivation section 10 is used to cultivate any one of the mushrooms, including dried mushrooms (or Lentinula edodes), king oyster mushrooms (or Pleurotus eryngii), whitening mushrooms (or Marmoreal mushrooms), oyster mushroom (or Phoenix oyster mushroom or Pleurotus ostreatus), and straw mushrooms (or Volvariella volvacea).

In the preferred embodiment of the present invention, the super-intensive breeding section 20 is used to breed any one of the aquatic products, including milkfish (or Chanos chanos), Taiwan sea bream (or snapper), tilapia (or Aquaculture of tilapia), perch, shrimp, eel, grouper, and blind tassel-fish (or four-finger threadfin or Eleutheronema tetradactylum).

In the preferred embodiment of the present invention, the non-toxic vegetable planting section 30 is used to cultivate artemisia lettuce and other vegetables.

In the preferred embodiment of the present invention, the poultry breeding section 40 is used to breed any one of the poultry, including chickens, ducks, and geese.

In the preferred embodiment of the present invention, the black soldier fly breeding section 60 is used to generate black soldier fly manure (or fertilizer).

In the preferred embodiment of the present invention, the black soldier flies in the black soldier fly breeding section 60 have larvae served as feedstuff of animals in the super-intensive breeding section 20 and the poultry breeding section 40.

In the preferred embodiment of the present invention, the mushroom cultivation section 10 occupies an area of 1,718 square meters, the super-intensive breeding section 20 occupies an area of 1,666 square meters, the non-toxic vegetable planting section 30 occupies an area of 2,502 square meters, the poultry breeding section 40 occupies an area of 89 square meters, the CAS cold chain section 50 occupies an area of 45 square meters, the black soldier fly breeding section 60 occupies an area of 104 square meters, the goods collection sales section 70 occupies an area of 2,000 square meters, and the green energy power generation section 90 occupies an area of 368 square meters.

In the preferred embodiment of the present invention, the CAS cold chain section 50 has an area proportion of one (1), the poultry breeding section 40 has an area proportion of 1.5 to 2.0, the black soldier fly breeding section 60 has an area proportion of 2.0 to 2.5, the green energy power generation section 90 has an area proportion of eight (8) to ten (10), the goods collection sales section 70 has an area proportion of forty (40) to forty five (45), the non-toxic vegetable planting section 30 has an area proportion of fifty five (55) to sixty (60), the super-intensive breeding section 20 has an area proportion of thirty five (35) to forty (40), and the mushroom cultivation section 10 has an area proportion of thirty five (35) to forty (40).

In the preferred embodiment of the present invention, the green energy power generation section 90 uses solar energy to generate the electricity. In addition, the excessive or surplus electric power from the green energy power generation section 90 is supplied to other parks or exported for sale.

In the black soldier fly breeding section 60, the black soldier flies eat the agricultural organic resources produced in the mushroom cultivation section 10, the super-intensive breeding section 20, the non-toxic vegetable planting section 30, the poultry breeding section 40, and the CAS cold chain section 50, to reduce the surplus in the mushroom cultivation section 10, the super-intensive breeding section 20, the non-toxic vegetable planting section 30, the poultry breeding section 40, and the CAS cold chain section 50. The agricultural organic resources include kitchen wastes, animal excrement, and organisms of animals and plants. In addition, after the larva of the black soldier fly grows up into a pupa gradually, the broken pupa emerges and transforms into a flying adult whose pupa shell can extract chitin or chitosan, which can be added to the feedstuff, and can also be used as a raw material for the industry, the agriculture, and the medical beauty. Moreover, after the black soldier flies eat the agricultural organic resources, the remaining residues produce the black soldier fly manure which can be used as excellent soil materials to improve the problems of soil acidification. Furthermore, the larvae of the black soldier flies are a good source of protein, and can be used as feedstuff for feeding the animals in the super-intensive breeding section 20 and the poultry breeding section 40. Thus, the black soldier fly breeding section 60 not only solves the problem of residues, but also will not produce new residues to construct a complete cycle of use.

In practice, the whole electric power consumed in the configuration system 100 of the green energy agricultural park is completely generated by the green energy power generation section 90, without needing an external power supply, to satisfy the requirement of self-sufficiency in power generation. In addition, the green energy power generation section 90 uses the solar energy to generate the electricity. Alternatively, the green energy power generation section 90 can also use other green energy generation method, such as wind power and hydraulic power, or other renewable energy sources, to generate the electricity.

It is appreciated that the ESG (environment, social, and governance) score contains three essential points, including environmental management, social responsibility, and corporate governance.

First of all, the present invention is expected to reduce carbon emission by 1,277 metric tons per year. Thus, the present invention is beneficial to the key index of environmental management in the ESG score, such as "Greenhouse Gas ISO14064, Carbon Neutral PAS2060" related to the carbon emission, "Water Carbon Footprint 14046, Carbon Footprint ISO/TS 14067" related to the carbon footprint of products, "Water Resource Efficiency Management ISO 46001" related to the water resource, and "Environmental Management ISO 14001, Environmental Management Guideline ISO 14004" related to the toxic substance emission and waste.

In addition, the present invention produces non-toxic agricultural products, processed food, and kitchen waste, finishes a waste environmental protection work, and regularly gives back to local farmers and to provide fertilizers, thereby fulfilling social responsibilities and earning benefits at the same time. Thus, the present invention is beneficial to the key index of social responsibility in the ESG score, such as "Quality Management System ISO 9001, Social Accountability Standard SA8000, and Social Responsibility Guideline ISO 26000" related to the safety and quality of products, and the opportunity of health and nutrition.

Further, the present invention can bring a huge outside income to the enterprise and help to make the sustainable development of the enterprise. Thus, the present invention is beneficial to the key index of governance in the ESG score, such as "Sustainable Accounting Standard SASB, Operational Continuity Management System ISO 22301" related to the stability and flexibility of the corporate financial system.

Accordingly, the configuration system 100 of the green energy agricultural park is self-sufficient in electricity, without needing an external power supply. In addition, the residues of agricultural products, aquatic products, and poultry products produced by the configuration system 100 of the green energy agricultural park can be eaten and resolved by the black soldier flies in the green energy agricultural park, and then completely recycled. Thus, the present invention satisfies requirements of the three essential points, including environmental management, social responsibility, and corporate governance, in the ESG score, and it is believed that the present invention will get a good evaluation in the ESG score, which is beneficial to the sustainable development of enterprises.

## Claims

1. A configuration system for a green energy agricultural park, comprising:
a mushroom cultivation section (10) for planting mushrooms;
a super-intensive breeding section (20) for breeding aquatic products;
a non-toxic vegetable planting section (30) for planting pesticide-free and organic vegetables;
a poultry breeding section (40) for breeding poultry;
a CAS cold chain section (50) for processing, packaging, and cold-chain storing agricultural organic matters produced in the mushroom cultivation section, the super-intensive breeding section, the non-toxic vegetable planting section, and the poultry breeding section;
a black soldier fly breeding section (60) for breeding black soldier flies which eat agricultural organic resources produced in the mushroom cultivation section, the super-intensive breeding section, the non-toxic vegetable planting section, the poultry breeding section, and the CAS cold chain section;
a goods collection sales section (70) for serving as a selling shop of agricultural products produced in the mushroom cultivation section, the super-intensive breeding section, the non-toxic vegetable planting section, the poultry breeding section, and the CAS cold chain section, and for collecting and transporting the agricultural products;
an electric vehicle charging section (80) for installing charging stations to charge electric cars and motorcycles; and
a green energy power generation section (90) for generating electricity and supplying an electric power to the mushroom cultivation section, the super-intensive breeding section, the non-toxic vegetable planting section, the poultry breeding section, the CAS cold chain section, the black soldier fly breeding section, the goods collection sales section, and the electric vehicle charging section.

2. The configuration system as claimed in claim 1, wherein the mushroom cultivation section is used to cultivate any one of the mushrooms, including dried mushrooms, king oyster mushrooms, whitening mushrooms, oyster mushroom, and straw mushrooms.

3. The configuration system as claimed in claim 1, wherein the super-intensive breeding section is used to breed any one of the aquatic products, including milkfish, Taiwan sea bream, tilapia, perch, shrimp, eel, grouper, and blind tassel-fish.

4. The configuration system as claimed in claim 1, wherein the non-toxic vegetable planting section is used to cultivate artemisia lettuce.

5. The configuration system as claimed in claim 1, wherein the poultry breeding section is used to breed any one of the poultry, including chickens, ducks, and geese.

6. The configuration system as claimed in claim 1, wherein the black soldier fly breeding section is used to generate black soldier fly manure.

7. The configuration system as claimed in claim 1, wherein the black soldier flies in the black soldier fly breeding section have larvae served as feedstuff of animals in the super-intensive breeding section and the poultry breeding section.

8. The configuration system as claimed in claim 1, wherein the mushroom cultivation section occupies an area of 1,718 square meters, the super-intensive breeding section occupies an area of 1,666 square meters, the non-toxic vegetable planting section occupies an area of 2,502 square meters, the poultry breeding section occupies an area of 89 square meters, the CAS cold chain section occupies an area of 45 square meters, the black soldier fly breeding section occupies an area of 104 square meters, the goods collection sales section occupies an area of 2,000 square meters, and the green energy power generation section occupies an area of 368 square meters.

9. The configuration system as claimed in claim 1, wherein the CAS cold chain section has an area proportion of one, the poultry breeding section has an area proportion of 1.5 to 2.0, the black soldier fly breeding section has an area proportion of 2.0 to 2.5, the green energy power generation section has an area proportion of eight to ten, the goods collection sales section has an area proportion of forty to forty five, the non-toxic vegetable planting section has an area proportion of fifty five to sixty, the super-intensive breeding section has an area proportion of thirty five to forty, and the mushroom cultivation section has an area proportion of thirty five to forty.

10. The configuration system as claimed in claim 1, wherein the green energy power generation section uses solar energy to generate the electricity.

## Amended claims

### Amended claims in accordance with Rule 137(2) EPC.

1. A configuration system for a green energy agricultural park, comprising:
a mushroom cultivation section (10) for planting mushrooms;
a super-intensive breeding section (20) for breeding aquatic products;
a non-toxic vegetable planting section (30) for planting pesticide-free and organic vegetables;
a poultry breeding section (40) for breeding poultry;
a CAS cold chain section (50) for processing, packaging, and cold-chain storing agricultural organic matters produced in the mushroom cultivation section, the super-intensive breeding section, the non-toxic vegetable planting section, and the poultry breeding section;
a black soldier fly breeding section (60) for breeding black soldier flies which eat agricultural organic resources produced in the mushroom cultivation section, the super-intensive breeding section, the non-toxic vegetable planting section, the poultry breeding section, and the CAS cold chain section, to reduce the surplus in the mushroom cultivation section, the super-intensive breeding section, the non-toxic vegetable planting section, the poultry breeding section, and the CAS cold chain section;
a goods collection sales section (70) for serving as a selling shop of agricultural products produced in the mushroom cultivation section, the super-intensive breeding section, the non-toxic vegetable planting section, the poultry breeding section, and the CAS cold chain section, and for collecting and transporting the agricultural products;
an electric vehicle charging section (80) for installing charging stations to charge electric cars and motorcycles; and
a green energy power generation section (90) for generating electricity and supplying an electric power to the mushroom cultivation section, the super-intensive breeding section, the non-toxic vegetable planting section, the poultry breeding section, the CAS cold chain section, the black soldier fly breeding section, the goods collection sales section, and the electric vehicle charging section;
wherein the black soldier fly breeding section is used to generate black soldier fly manure used as soil materials to improve the problems of soil acidification;
wherein the black soldier flies in the black soldier fly breeding section have larvae served as feedstuff of animals in the super-intensive breeding section and the poultry breeding section and used as a raw material;
wherein the black soldier fly breeding section constructs a complete cycle of use;
wherein the mushroom cultivation section occupies an area of 1,718 square meters, the super-intensive breeding section occupies an area of 1,666 square meters, the non-toxic vegetable planting section occupies an area of 2,502 square meters, the poultry breeding section occupies an area of 89 square meters, the CAS cold chain section occupies an area of 45 square meters, the black soldier fly breeding section occupies an area of 104 square meters, the goods collection sales section occupies an area of 2,000 square meters, and the green energy power generation section occupies an area of 368 square meters;
wherein the CAS cold chain section has an area proportion of one, the poultry breeding section has an area proportion of 1.5 to 2.0, the black soldier fly breeding section has an area proportion of 2.0 to 2.5, the green energy power generation section has an area proportion of eight to ten, the goods collection sales section has an area proportion of forty to forty five, the non-toxic vegetable planting section has an area proportion of fifty five to sixty, the super-intensive breeding section has an area proportion of thirty five to forty, and the mushroom cultivation section has an area proportion of thirty five to forty;
wherein the whole electric power consumed in the configuration system of the green energy agricultural park is completely generated by the green energy power generation section, without needing an external power supply, to satisfy the requirement of self-sufficiency in power generation.

2. The configuration system as claimed in claim 1, wherein the mushroom cultivation section is used to cultivate any one of the mushrooms, including dried mushrooms, king oyster mushrooms, whitening mushrooms, oyster mushroom, and straw mushrooms.

3. The configuration system as claimed in claim 1, wherein the super-intensive breeding section is used to breed any one of the aquatic products, including milkfish, Taiwan sea bream, tilapia, perch, shrimp, eel, grouper, and blind tassel-fish.

4. The configuration system as claimed in claim 1, wherein the non-toxic vegetable planting section is used to cultivate artemisia lettuce.

5. The configuration system as claimed in claim 1, wherein the poultry breeding section is used to breed any one of the poultry, including chickens, ducks, and geese.

6. The configuration system as claimed in claim 1, wherein the green energy power generation section uses solar energy to generate the electricity.
